# EUROPÄISCHE PATENTSCHRIFT

(11) **EP 0 802 174 B1**
(45) Veröffentlichungstag und Bekanntmachung des Hinweises auf die Patenterteilung: **16.08.2000**
(21) Anmeldenummer: 97103939.1
(22) Anmeldetag: 10.03.1997
(51) Int. Cl.: C07C 29/149, C07C 31/133

(54) **Verfahren zur Herstellung von Hydroxymethylcyclopropan**
Process for the preparation of hydroxymethylcyclopropane
Procédé pour la préparation d'hydroxyméthylcyclopropane

(30) Priorität: 21.03.1996 DE 19611142
(43) Veröffentlichungstag der Anmeldung: 22.10.1997
(73) Patentinhaber: BAYER AG, 51368 Leverkusen (DE)
(72) Erfinder: Darsow, Gerhard, Dr., 47804 Krefeld (DE); Frohn, Lutz, Dr., 40699 Erkrath (DE); Langer, Reinhard, Dr., 47800 Krefeld (DE)

(56) Entgegenhaltungen:
- EP-A- 0 523 818
- DE-A- 19 505 939
- US-A- 4 720 597

## Beschreibung

Die vorliegende Erfindung betrifft ein Verfahren zur Herstellung von Hydroxymethyl-cyclopropan durch katalytische Hydrierung von Alkylestern der Cyclopropancarbonsäure mit Wasserstoff unter Einsatz eines Festbettkatalysators.

Hydroxymethyl-cyclopropan findet Verwendung zur Herstellung von pharmazeutischen Produkten und Pflanzenschutzmitteln.

Es ist bekannt, Hydroxymethyl-cyclopropan durch katalytische Hydrierung von Cyclopropancarbonsäureestern herzustellen (siehe US-PS 4 720 597). Für dieses Verfahren wird ein Zink-Chrom-Katalysator eingesetzt, der wegen seines Chromgehaltes besonders toxisch ist, nur eine kurze Lebensdauer aufweist und Reaktionstemperaturen von über 200°C, beispielsweise von 250 bis 300°C benötigt.

Es besteht daher der Wunsch nach einem Katalysator zur Herstellung von Hydroxymethyl-cyclopropan durch Hydrierung von Cyclopropancarbonsäureestern, der weniger toxisch ist, eine erhöhte Lebensdauer aufweist und bei geringeren Reaktionstemperaturen angewendet werden kann.

Es wurde jetzt gefunden, daß die genannten Anforderungen durch einen reduzierten stückigen Katalysator erfüllt werden, der durch Verpressen eines Gemisches von pulverigen Kupfer-, Zink- und Aluminium-Oxiden und anschließende Reduktion erhältlich ist. Vorzugsweise enthält das pulvrige Oxidgemisch zusätzlich mindestens ein Oxid eines Metalls der Eisengruppe des Periodensystems der Elemente (Mendelejew).

Es wurde somit ein Verfahren zur Herstellung von Hydroxymethyl-cyclopropan aus Cyclopropancarbonsäure-alkylestern gefunden, das dadurch gekennzeichnet ist, daß man Cyclopropancarbonsäure-alkylester mit überschüssigem Wasserstoff bei Reaktionstemperaturen von 100 bis 190°C und einem Wasserstoffdruck von 30 bis 400 bar in Gegenwart eines reduzierten, stückigen Katalysators hydriert, der erhältlich ist durch Verpressen eines Gemisches von pulvrigen Kupfer-, Zink- und Aluminium-Oxiden und anschließende Reduktion.

Zum Einsatz in das erfindungsgemäße Verfahren geeignete Cyclopropancarbonsäure-alkylester sind z.B. solche, in denen der Alkylrest 1 bis 10 C-Atome aufweist. Beispiele für solche Alkylreste sind Methyl, Ethyl, Propyl, Butyl, Isobutyl, Pentyl, Hexyl, Octyl und Decyl. Bevorzugt sind Methyl, Ethyl, Propyl, Butyl, Isobutyl und Hexyl. Besonders bevorzugt sind Methyl, Ethyl, Propyl, Butyl und Isobutyl.

Erfindungsgemäß wird mit einem Wasserstoffüberschuß gearbeitet. Die Wasserstoffmenge kann z.B. das 10- bis 60-fache der stöchiometrisch erforderlichen Menge sein.

Der Druckbereich für das erfindungsgemäße Verfahren liegt im Bereich von 30 bis 400 bar. Bevorzugt sind 120 bis 350 bar, besonders bevorzugt 250 bis 320 bar.

Erfindungsgemäß wird bei einer Temperatur von 100 bis 190°C gearbeitet. Bevorzugt liegt die Temperatur im Bereich von 130 bis 180°C.

Es ist möglich, das erfindungsgemäße Verfahren unter Zusatz eines Lösungsmittels oder Lösemittelgemisches durchzuführen, wobei für katalytische Hydrierungen geläufige Lösungsmittel infrage kommen, z.B. aliphatische Alkohole. Da die Anwesenheit von Lösungsmitteln die destillative Aufarbeitung des Reaktionsgemisches erschwert, wird aber bevorzugt ohne Zusatz von Lösungsmitteln gearbeitet.

Das erfindungsgemäße Verfahren kann z.B. in der Gas- oder Flüssigphase durchgeführt werden. Das Arbeiten in der flüssigen Phase, z.B. in der sogenannten Rieselphase, ist bevorzugt.

Zur Hydrierung von Estern werden vielfach diskontinuierliche Verfahren (Batch-Prozeß) angewendet, bei denen pulverförmige Katalysatoren in suspensierter Form zum Einsatz kommen. Im Gegensatz dazu kann das erfindungsgemäße Verfahren mit einem stückigen Katalysator und mit hohen Standzeiten des Katalysators kontinuierlich durchgeführt werden. Hierdurch werden die unvermeidlichen Handhabungsprobleme vermieden, die pulverförmige Katalysatoren aufweisen, z.B. die Schwierigkeit, pulverförmige Katalysatoren gezielt und gleichmäßig zu aktivieren, die Schwierigkeit, sie mit Schlammpumpen umzuwälzen, und die Schwierigkeit, sie vom Reaktionsprodukt vollständig abzutrennen. Schlammpumpen sind sehr verschleißanfällig, weil sie einer hohen mechanischen Beanspruchung unterliegen. Die vollständige Entfernung pulverförmiger Katalysatoren ist aufwendig, weil sie eine Grob- und eine Feinfiltration erfordert und die Filter für wechselseitigen Betrieb und Reinigung mindestens jeweils zweifach vorhanden sein müssen. Außerdem ist die Gefahr groß, daß pulverförmige Katalysatoren infolge der Umwälzung und Filtration schnell ihre Aktivität verlieren, was hohe Katalysatorverbräuche bzw. kurze Katalysatorlaufzeiten zur Folge hat. Im Gegensatz zu diesen aufgezeigten Schwierigkeiten sind einfindungsgemäß einzusetzende stückige Katalysatoren besser handhabbar und weisen eine hohe Aktivität auf, die über Zeiträume von z.B. bis zu mehreren Jahren erhalten bleibt. Der letztere Vorteil ist besonders bedeutsam, da bei solchen langen Katalysatorstandzeiten nur sehr selten ein Katalysatorwechsel notwendig wird, der auch bei im Festbett angeordneten Katalysatoren aufwendig ist.

Der erfindungsgemäß einzusetzende stückige Katalysator ist erhältlich aus einem Gemisch von pulvrigen Kupfer-, Zink- und Aluminiumoxiden. In dem pulvrigen Oxidgemisch kann der Kupferanteil z.B. 40 bis 60 Gew.-%, der Zinkanteil z.B. 15 bis 30 Gew.-% und der Aluminiumanteil z.B. 0,2 bis 6 Gew.-% betragen, wobei diese Angaben sich auf die Gesamtmenge des pulverförmigen Oxidgemisches beziehen und der Rest zu 100 Gew.-% Sauerstoff darstellt. Das pulverförmige Oxidgemisch kann die Oxide z.B. in einer mittleren Korngröße von weniger als 200 µm enthalten. Von den Kupferoxiden ist Kupfer(II)-oxid bevorzugt. Ein aus solchen Gemischen durch Verpressen und Reduktion hergestellter Katalysator kann in die erfindungsgemäße Hydrierung ohne weitere Zusätze eingesetzt werden.

Vorteilhafterweise enthält das pulverförmige Gemisch aber zusätzlich mindestens ein Oxid eines Metalls der Eisengruppe des Periodensystems der Elemente (Mendelejew). Bevorzugt hierfür sind Oxide von Eisen, Kobalt und/oder Nickel, in denen die Metalle die Oxidationsstufen +2 und/oder +3 aufweisen können. Besonders bevorzugt ist hier Eisen(III)-oxid. Oxide von Eisen, Kobalt und Nickel, bevorzugt von Eisen und Kobalt, können sowohl einzeln als auch im Gemisch mit anderen Oxiden der genannten Metalle eingesetzt werden. Die Gesamtmenge von Oxiden von Metallen der Eisengruppe im pulverförmigen Oxidgemisch kann z.B. 0,1 bis 1,5 Gew.-% des gesamten Oxidpulvers betragen. Bevorzugt sind 0,05 bis 1,0, insbesondere 0,1 bis 0,5 Gew.-%. Für den Fall des Einsatzes mehrerer Oxide von Metallen der Eisengruppe kann jedes einzelne dieser Oxide z.B. in einer Menge vorliegen, die nicht kleiner ist als 20 Gew.-% und nicht größer als 80 Gew.-%. der Gesamtmenge an Oxiden von Metallen der Eisengruppe.

Es ist vorteilhaft, für das erfindungsgemäße Verfahren Katalysatoren einzusetzen, die möglichst frei von Alkali- und Erdalkaliverbindungen sind. Solche Verunreinigungen sollten allenfalls zu weniger 0,1 Gew.-%, bezogen auf die Gesamtmenge des Gemisches pulveriger Oxide, vorhanden sein.

Die Herstellung eines stückigen Katalysators aus dem Gemisch pulverförmiger Oxide kann nach an sich gebräuchlichen Methoden durch Verpressen erfolgen, beispielsweise unter hohem Druck auf Tablettier- oder Pelletiermaschinen, wobei gegebenenfalls zur Verbesserung des Haftvermögens der Metalloxidpartikel Graphit und/oder Klebstoffe z.B. in Mengen bis zu 3 Gew.-%, bezogen auf das Gesamtgewicht der zu verpressenden Bestandteile, zugesetzt werden können. Die verpreßten Katalysatoren können z.B. als Tabletten, Kugeln oder Granulate vorliegen mit Abmessungen im Bereich von 2 bis 10 mm, bevorzugt 3 bis 7 mm. Die stückigen, insbesondere als Tabletten vorliegenden Formkörper, können zur Vergrößerung der äußeren Oberfläche mit einer Durchbohrung versehen sein. Solche Formkörper haben makroskopisch betrachtet eine glatte Oberfläche.

Die stückigen Katalysatoren werden so verpreßt, daß sie auf der Formkörperoberfläche eine Druckfestigkeit von z.B. 50 bis 200 N, bevorzugt von 75 bis 150 N, aufweisen. Die stückigen Katalysatoren besitzen ferner eine innere Oberfläche von z.B. 10 bis 90 m²/g, bevozugt 30 bis 80 m²/g. Die Druckfestigkeit der stückigen Katalysatoren kann z.B. nach DIN 50 106 bestimmt werden, die innere Oberfläche z.B. nach Analyt. Chem. 30, 1387-1392 (1958) oder nach S.J. Gregg und S.W. Sing, Adsorption, Surface Area and Porosity, London 1982, Kap. 2 und 6 erfolgen.

Die erfindungsgemäß als Hydrierkatalysatoren einzusetzenden Formkörper aus verpreßten Pulvern von Kupfer-, Zink-, Aluminium- und gegebenenfalls weiteren Oxiden müssen vor ihrem Einsatz reduziert werden. Dies geschieht am einfachsten durch eine Behandlung mit Wasserstoff, z.B. bei 180 bis 280°C. Es ist vorteilhaft, zu Beginn der Behandlung ein Gemisch aus beispielsweise 10 bis 15 Vol.-% Wasserstoff und 90 bis 85 Vol.-% Inertgas (z.B. Stickstoff) einzusetzen und im Verlaufe der Behandlung den Inertgasanteil bis auf Null zu vermindern. Eine solche Behandlung kann z.B. in einem Zeitraum von 10 bis 35 Stunden durchgeführt werden. Die Behandlung kann dann beendet werden, wenn kein Wasserstoff mehr aufgenommen und infolgedessen kein Reaktionswasser mehr gebildet wird.

Der zu hydrierende Ester kann im Hydrierreaktor von unten nach oben oder von oben nach unten strömen. Es ist vorteilhaft, den Ester gasförmig oder flüssig von oben nach unten über den Katalysator fließen zu lassen (Rieselphase). Dabei kann der zu hydrierende Ester entweder gemeinsam mit dem separat eingeführten oder vorher zugemischten Wasserstoff über den Katalysator strömen (= Gleichstromverfahren) oder aber dem Wasserstoff entegegengeführt werden (= Gegenstromverfahren).

Der Hydrierreaktor kann z.B. ein Hochdruckrohr aus Stahl sein, das mit stückigem Katalysator ganz oder teilweise gefüllt ist, wobei bei größeren Rohrquerschnitten auch die Anwendung der stückigen Katalysatoren auf Horden (Drahtkörbe oder ähnliches) in Betracht zu ziehen ist. Man kann z.B. auch Hochdruckrohrbündel innerhalb eines gemeinsamen Mantels anwenden, wobei die Einzelrohre wiederum mit den stückigen Katalysatoren ganz oder teilweise gefüllt sind.

Die stündliche Katalysatorbelastung kann beim erfindungsgemäßen Verfahren z.B. bei 200 bis 600 g Ester pro Liter Katalysator liegen. Unter den genannten sonstigen Reaktionsbedingungen sind mit den erfindungsgemäß einzusetzenden Katalysatoren hohe Standzeiten von beispielsweise 8 000 bis 16 000 Stunden zu erreichen, was sie für kontinuierliche Verfahren besonders geeignet macht. Mit den in der US-PS 4 720 597 beschriebenen Katalysatoren beträgt die Standzeit im allgemeinen nur 3.000 bis 4.000 Stunden.

Das den Hydrierreaktor verlassende Reaktionsgemisch enthält nach der Entspannung, bei welcher man den überschüssigen Wasserstoff abfangen und nach erfolgter Kompression und Ergänzung des verbrauchten Wasserstoffs erneut zum Einsatz bringen kann, hauptsächlich Hydroxymethylcyclopropan und den Alkohol, der dem Alkoholteil des eingesetzten Cyclopropancarbonsäurealkylesters entspricht. Diese beiden Komponenten können destillativ getrennt werden. Das erzeugte Hydroxymethyl-cyclopropan kann nach der destillativen Abtrennung in einer Reinheit bis zu 99,9 Gew.-% erhalten werden. Es ist in dieser Qualität direkt für weitere Umsetzungen geeignet.

### Beispiele

### Beispiel 1

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstoff-frei gespült worden war, wurde mit 1,4 l eines durch Tablettierung von Pulvern einer mittleren Korngröße von weniger als 200 µm von Kupfer(II)-, Zink-, Aluminium- und Eisen(III)-oxiden hergestellten Hydrierkatalysators gefüllt. Der Kupfergehalt der Tabletten lag bei 42 Gew.-%, der Zinkgehalt bei 17 Gew.-%, der Aluminiumgehalt bei 2,0 Gew.-% und der Eisengehalt bei 0,2 Gew.-%. Die Tabletten hatten eine Höhe und einen Durchmesser von jeweils 5 mm, eine Druckfestigkeit von 125 N auf der Zylindermantelfläche und eine innere Oberfläche von 68 m²/g.

Zur Aktivierung durch Reduktion wurden die Tabletten zunächst 6 Stunden lang im Stickstoffstrom getrocknet (Temperatur: max. 200°C; Stoffmenge: 5 Nm³/h). Die eigentliche Aktivierung erfolgte beim einem Stickstoffdruck von 200 bar und einer Temperatur zwischen 180 und 280°C, wobei dem Inertgas allmählich Wasserstoff zugemischt wurde, dessen Zumischungsanteil in der Anfangsphase 15 Vol.-% nicht überstieg. Innerhalb von 24 Stunden wurde der Stickstoffanteil der Gasmischung mehr und mehr vermindert, bis schließlich reiner Wasserstoff den Reaktor durchströmte. Die Aktivierung war beendet, als kein Reaktionswasser mehr gebildet wurde, was mit einem nachgeschalteten Abscheider kontrolliert wurde.

Nach der Aktivierung des Hydrierkatalysators wurde der Wasserstoffdruck im Reaktorsystem auf 300 bar erhöht. Anschließend wurden stündlich 280 g Cyclopropancarbonsäure-methylester, gemeinsam mit 2 Nm³ Wasserstoff unter einem Druck von 300 bar durch das Hochdruckrohr gepumpt, wobei der Cyclopropancarbonsäure-methylester vor Eintritt in das Hochdruckrohr in einem vorgeschalteten elektrisch beheizten Wärmeaustauscher auf eine Temperatur von 160°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur von unter 60°C abgekühlt und in einem Gasabscheider vom überschüssigen Wasserstoff, der wieder in das Hydriersystem zurückgeführt wurde, abgetrennt. Nach weiterer Abkühlung auf eine Temperatur unter 30°C und Entspannung auf Normaldruck wurde das Reaktionsprodukt gaschromatographisch untersucht.

Der Umsatz an Cyclopropancarbonsäure-methylester betrug 99 %, die Selektivität zu Hydroxymethyl-cyclopropan 98 %. Der Katalysator war nach einer Laufzeit von 7.600 Stunden noch hochwirksam.

### Beispiel 2

Es wurde verfahren wie in Beispiel 1, jedoch wurde ein Katalysator verwendet, der nur 0,1 Gew.-% Eisen enthielt. Die Tabletten wiesen die gleichen Abmessungen, Druckfestigkeiten und inneren Oberflächen wie die in Beispiel 1 beschriebenen auf. Statt 280 g Cyclopropancarbonsäuremethylester wurden in diesem Fall stündlich 320 g Cyclopropancarbonsäureethylester eingesetzt. Gemäß gaschromatographischer Untersuchung betrug der Umsatz von Cyclopropancarbonsäureethylester über 99,5 % und die Selektivität für die Bildung von Hydroxymethylcyclopropan 96 %. Der Katalysator hatte nach einer Laufzeit von 5.800 Stunden noch nichts von seiner Aktivität eingebüßt.

### Beispiel 3

Ein Hochdruckreaktor wie in Beispiel 1 wurde unter Inertgas mit 1,4 l eines durch Tablettierung von Pulvern von Kupfer(II)-, Zink- und Aluminiumoxid mit einer mittleren Korngröße von weniger als 200 µm hergestellten Hydrierungskatalysators gefüllt. Der Kupfergehalt der Tabletten lag bei 51 Gew.-%, der Zinkgehalt bei 19 Gew.,-% und der Aluminiumgehalt bei 0,5 Gew.-%. Die Tabletten hatten eine Höhe und einen Durchmesser von jeweils 3 mm, eine Druckfestigkeit von 81 N auf die Zylindermantelfläche und eine innere Oberfläche von 58 m²/g.

Nach der Aktivierung der Tabletten durch Reduktion wie in Beispiel 1 wurde der Wasserstoffdruck auf 300 bar erhöht. Anschließend wurden stündlich 280 g Cyclopropancarbonsäure-isobutylester, gemeinsam mit 1,5 Nm³ Wasserstoff unter einem Druck von 300 bar kontinuierlich durch das Hochdruckrohr gepumpt, wobei der Cyclopropancarbonsäure-isobutylester vor Eintritt in das Hochdruckrohr auf eine Temperatur von 160°C erhitzt wurde.

Das das Reaktionsrohr verlassende Reaktionsprodukt wurde, wie in Beispiel 1 beschrieben, aufgearbeitet und gaschromatographisch untersucht.

Der Umsatz an Cyclopropancarbonsäure-isobutylester betrug 99,5 %, die Selektivität für die Bildung von Hydroxymethyl-cyclopropan 98 %. Der Katalysator war nach einer Laufzeit von 6.200 Stunden noch hochwirksam.

## Patentansprüche

1. Verfahren zur Herstellung von Hydroxymethyl-cyclopropan aus Cyclopropancarbonsäure-alkylestern, dadurch gekennzeichnet, daß man Cyclopropancarbonsäure-alkylester mit überschüssigem Wasserstoff bei Reaktionstemperaturen von 100 bis 190°C und einem Wasserstoffdruck von 30 bis 400 bar in Gegenwart eines reduzierten stückigen Katalysators hydriert, der erhältlich ist durch Verpressen eines Gemisches von pulvrigen Kupfer-, Zink- und Aluminiumoxiden und anschließende Reduktion.

2. Verfahren nach Anspruch 1, dadurch gekennzeichnet, daß im eingesetzten Cyclopropancarbonsäure-alkylester der Alkylrest 1 bis 10 C-Atome aufweist.

3. Verfahren nach Ansprüchen 1 und 2, dadurch gekennzeichnet, daß die 10bis 60fache Menge der stöchiometrisch erforderlichen Menge Wasserstoff eingesetzt wird.

4. Verfahren nach Ansprüchen 1 bis 3, dadurch gekennzeichnet, daß man es bei 120 bis 350 bar und 130 bis 180°C durchführt.

5. Verfahren nach Ansprüchen 1 bis 4, dadurch gekennzeichnet, daß man es in der flüssigen Phase oder in Rieselphase durchführt.

6. Verfahren nach Ansprüchen 1 bis 5, dadurch gekennzeichnet, daß das Gemisch pulvriger Kupfer-, Zink- und Aluminiumoxide zusätzlich mindestens ein Oxid eines Metalls der Eisengruppe des Periodensystems der Elemente (Mendelejew) enthält.

7. Verfahren nach Ansprüchen 1 bis 6, dadurch gekennzeichnet, daß das Gemisch pulvriger Oxide einen Kupferanteil von 40 bis 60 Gew.-%, einen Zinkanteil von 15 bis 30 Gew.-%, einen Aluminiumanteil von 0,2 bis 6 Gew.-% und gegebenenfalls Oxide von Metallen der Eisengruppe bis zu 1,5 Gew.-% enthält.

8. Verfahren nach Ansprüchen 1 bis 7, dadurch gekennzeichnet, daß die verpreßten Katalysatoren Abmessungen im Bereich von 2 bis 10 mm, eine Druckfestigkeit von 50 bis 200 N auf der Formkörperoberfläche und eine innere Oberfläche von 10 bis 90 m²/g aufweisen.

9. Verfahren nach Ansprüchen 1 bis 8, dadurch gekennzeichnet, daß der Katalysator vor Inbetriebnahme reduziert wird durch eine Behandlung mit Wasserstoff bei 180 bis 280°C, wobei man vor Beginn der Behandlung ein Gemisch aus 10 bis 15 Vol.-% Wasserstoff und 90 bis 85 Vol.-% Inertgas einsetzt und im Verlaufe der Behandlung den Inertgasanteil bis auf 0 vermindert.

10. Verfahren nach Ansprüchen 1 bis 9, dadurch gekennzeichnet, daß man aus den Reaktor verlassende Reaktionsgemisch entspannt, überschüssigen Wasserstoff abfängt und erneut zum Einsatz bringt und das gebildete Hydroxymethylcyclopropan und den gebildeten Alkohol destillativ trennt.

## Claims

1. Process for the preparation of hydroxymethylcyclopropane from alkyl cyclopropanecarboxylates, characterised in that alkyl cyclopropanecarboxylates are hydrogenated with excess hydrogen at reaction temperatures of 100°C to 190°C and at a hydrogen pressure of 30 to 400 bar in the presence of a reduced catalyst in the form of lumps, which is obtainable by pressing a mixture of pulverulent oxides of copper, of zinc and of aluminium and subsequent reduction.

2. Process according to claim 1, characterised in that the alkyl group in the alkyl cyclopropanecarboxylate used has 1 to 10 C atoms.

3. Process according to claims 1 and 2, characterised in that 10 to 60 times the amount of the stoichiometrically required quantity of hydrogen is used.

4. Process according to claims 1 to 3, characterised in that it is carried out at 120 to 350 bar and at 130°C to 180°C.

5. Process according to claims 1 to 4, characterised in that it is carried out in the liquid phase or in trickling phase.

6. Process according to claims 1 to 5, characterised in that the mixture of pulverulent oxides of copper, of zinc and of aluminium contains in addition at least one oxide of a metal of the iron group of the periodic system of elements (Mendeleev).

7. Process according to claims 1 to 6, characterised in that the mixture of pulverulent oxides has a copper content of 40 to 60 wt.%, a zinc content of 15 to 30 wt.%, an aluminium content of 0.2 to 6 wt.% and optionally contains up to 1.5 wt.% of oxides of metals of the iron group.

8. Process according to claims 1 to 7, characterised in that the pressed catalysts have dimensions within the range of 2 to 10 mm, a compressive strength of 50 to 200 N on the surface of the moulding and an internal surface area of 10 to 90 m²/g.

9. Process according to claims 1 to 8, characterised in that, prior to being put into operation, the catalyst is reduced by a treatment with hydrogen at 180°C to 280°C, a mixture of 10 to 15 vol.% hydrogen and 90 to 85 vol.% inert gas being used prior to commencement of the treatment and the proportion of inert gas being decreased to 0 in the course of the treatment.

10. Process according to claims 1 to 9, characterised in that the reaction mixture leaving the reactor is released, excess hydrogen is collected and again reused and the hydroxymethylcyclopropane formed and the alcohol formed are separated by distillation.

## Revendications

1. Procédé de préparation de l'hydroxyméthylcyclopropane à partir d'esters alkyliques de l'acide cyclopropanecarboxylique, caractérisé en ce que l'on hydrogène le cyclopropanecarboxylate d'alkyle avec un excès d'hydrogène à des températures de réaction de 100 à 190°C sous une pression d'hydrogène de 30 à 400 bar en présence d'un catalyseur à l'état de morceaux individuels et réduit, qu'on obtient par compression d'un mélange d'oxydes de cuivre, de zinc et d'aluminium en poudre suivie d'une réduction.

2. Procédé selon la revendication 1, caractérisé en ce que le groupe alkyle du cyclopropanecarboxylate d'alkyle contient 1 à 10 atomes de carbone.

3. Procédé selon les revendications 1 et 2, caractérisé en ce que l'on utilise l'hydrogène en quantité de 10 à 60 fois la quantité théorique nécessaire.

4. Procédé selon les revendications 1 à 3, caractérisé en ce que l'on opère sous une pression de 120 à 350 bar à une température de 130 à 180°C.

5. Procédé selon les revendications 1 à 4, caractérisé en ce que l'on opère en phase liquide ou en phase ruisselante.

6. Procédé selon les revendications 1 à 5, caractérisé en ce que le mélange d'oxydes de cuivre, de zinc et d'aluminium en poudre contient en outre au moins un oxyde d'un métal du groupe du fer de la Classification Périodique des Eléments (Mendelejeff).

7. Procédé selon les revendications 1 à 6, caractérisé en ce que le mélange d'oxydes en poudre est à une teneur en cuivre de 40 à 60 % en poids, une teneur en zinc de 15 à 30 % en poids, une teneur en aluminium de 0,2 à 6 % en poids et le cas échéant une teneur en oxydes de métaux ou groupes du fer allant jusqu'à 1,5 % en poids.

8. Procédé selon les revendications 1 à 7, caractérisé en ce que les catalyseurs comprimés ont des dimensions dans l'intervalle de 2 à 10 mm, une résistance à la compression de 50 à 200 N à la surface des objets moulés et une surface interne de 10 à 90 m²/g.

9. Procédé selon les revendications 1 à 8, caractérisé en ce que le catalyseur, avant mise en service, est réduit par traitement à l'aide d'hydrogène à des températures de 180 à 280°C, avec, au début du traitement, un mélange de 10 à 15 % en volume d'hydrogène et de 90 à 85 % en volume de gaz inerte, la proportion de gaz inerte étant diminuée jusqu'à élimination complète au cours du traitement.

10. Procédé selon les revendications 1 à 9, caractérisé en ce que le mélange de réaction quittant le réacteur est détendu, l'excès d'hydrogène est recueilli et renvoyé à l'utilisation et l'hydroxyméthylcyclopropane formé est séparé de l'alcool libéré par distillation.
